# EUROPEAN PATENT SPECIFICATION

(11) **EP 2 087 918 B1**
(45) Date of publication and mention of the grant of the patent: **18.01.2012**
(21) Application number: 09001640.3
(22) Date of filing: 05.02.2009
(51) Int. Cl.: A61M 15/00

(54) **Blister piercer**
Blisterlochstanze
Perceur de coque

(30) Priority: 05.02.2008 GB 0802030
(43) Date of publication of application: 12.08.2009
(73) Proprietor: Boehringer Ingelheim Pharma GmbH & Co. KG, 55216 Ingelheim am Rhein (DE)
(72) Inventor: Dunne, Stephen T., Stowmarket Suffolk, IP14 3AE (GB)
(74) Representative: Gesthuysen, von Rohr & Eggert

(56) References cited:
- EP-A- 1 106 196
- US-A- 3 921 637
- US-A- 3 991 761
- US-A1- 2007 221 216

## Description

### Backgroud of the Invention:

This invention is concerned with dry powder inhalers for the delivery of drugs to the lungs.

Many dry powder inhalers are on the market or have been proposed. There are two main types; passive and active. In passive devices all the energy required for deagglomeration the powder and transferring the powder to the lungs is provided by the patient. Most powder inhalers are of the passive type where the powder is inhaled by the patient without the aid of a secondary energy source.

Dry powder inhalers are subdivided into single dose devices and multi dose devices. Multi dose inhalers are further subdivided into pre metered types where the doses are stored individually in the device and metering devices where the powder dose is metered in the device.

Multi dose pre metered devices have the advantage that the single doses are metered under strict factory conditions and the powder can quite easily be isolated from the atmosphere. In many applications the active drug powder is mixed with a carrier such as lactose which tends to absorb humidity from the atmosphere which makes it stick together and difficult to deagglomerate.

Many pre metered devices use sealed blisters to store the powder. Most devices either peal the blister lid to access the powder or punch the lidding to blow the powder out. The disadvantage of these methods is that all powder is delivered every time.

EP 1 106 196 A2 relates to a powder inhaler comprising a mouthpiece, a holder mounting portion and a holder rotatably mounted to the holder mounting portion and holding thereon a blister pack. A piercing tool is provided for piercing an inflow hole and an outflow hole into a blister of the blister pack. The piercing tool has a pair of parallel pins spaced apart from each other.

US 3,991,761 discloses an inhaler for powdered medicaments wherein the powder is contained in a capsule. The inhaler comprises a capsule chamber, wherein the chamber has an elongated recess in a bottom wall in which the capsule rests when the inhaler is not in use. A piercing device is provided having movable push buttons. Each of the push bottoms supports four elongated metal spikes. The spikes or pins are coaxial with the recess so that the capsule is perforated at its longitudinal ends.

US 2007/0221216 A1 relates to an inhaler having a chamber for receiving a capsule shaped medication container. An air passage connecting the container chamber with external air. A mouthpiece is axially engageable with an inhaler body and a hollow needle communicating with the mouthpiece. In one embodiment the hollow needle and one or more secondary hollow needles are provided, wherein the secondary needles penetrate a one longitudinal end and the main needle penetrates both longitudinal ends of the medication container.

US 3,921,637 discloses an inhaler for powdered medicaments. The inhaler comprises a housing having a chamber for receiving a capsule. Two longitudinal ends of the capsule are punctured using a first needle and then a second needle.

### Summary of the Invention:

The present invention is a blister piercing mechanism that ensures all (or nearly all) of the powder medicament is delivered every time from ever blister. The invention

is described in claim 1.

Preferably the hole or holes made by the first piercer have a total cross sectional area of between 10 to 50 square millimetres. The holes or holes may be position anywhere in the lidding. One hole is sufficient in most cases and maybe in the edges or centre of the lidding.

Because the blister body is relatively tough the hole(s) made by the second piercer in the body are preferable made by sharp pins or needles. In the preferred embodiment a number of holes are made with sharp pins. The pins preferably have a diameter between 0.1 and 2.0 mm and more preferably between 0.3 and 1.2 mm. The total cross sectional area of the holes is preferably between 10 to 50 square millimetres. To ensure that all or most of the powder is delivered the second piercer is arranged so that numerous holes are made in the blister body and evenly spaced out over the entire blister body. The number of holes is at least 4 and may vary, preferably up to 50 holes (more preferably 5 to 20). This is the key advantage to this invention. Because the air drawn (passive inhaler) or forced (active inhaler) into the blister is evenly distributed over the entire blister it is easier to deliver all the powder contained within. If solid pins are used the piercers needs to be disengaged from the body to allow air or gas flow into the blisters. Alternatively instead of holes hollow needles or other such shape may be used both in the blister lid and blister body. In this way the piercers may be kept in their punching positions.

The blister may be a circular, oval, rectangular, square or any other cross section viewed from the top facing the blister lid. The blister may have sharp corners or rounded corners. The blister may be manufactured in any material preferably plastic. Preferably the blister lid is made from a rigid material so that its from is not distorted when piercing. The blister may have a humidity protective barrier such as a thin layer of aluminium. The blister lid may be manafactured in any material preferably plastic. The blister lid may have a humidity protective barrier such as a thin layer of aluminium. Preferably the blister lid is made from a rupturable material rupture able with a relatively blunt piercer.

The mass of each dose of powder may be between 1 and 100 mg and more preferably between 2 and 50 mg. The powder to be dispensed maybe a pure micronised drug or a mixture of drugs or a mixture of a micronised drug or drugs with a carrier such as lactose or a mixture of carriers such as lactose.

The blisters may be single blisters to be inserted by the user into the device one by one or may be on a disc, a strip or individually stored in a magazine or other container in a multi dose device.

The gas or air flow used to release the powder from the blister may be generated by the user in a passive system or by mechanical means such as a gas pump or compressed gas such as 134a in an active system.

### Brief Description of the Drawings:

In order to further describe the invention drawings will be used. Not all possible embodiments are shown. It must be understood that other embodiments are possible based on the invention.

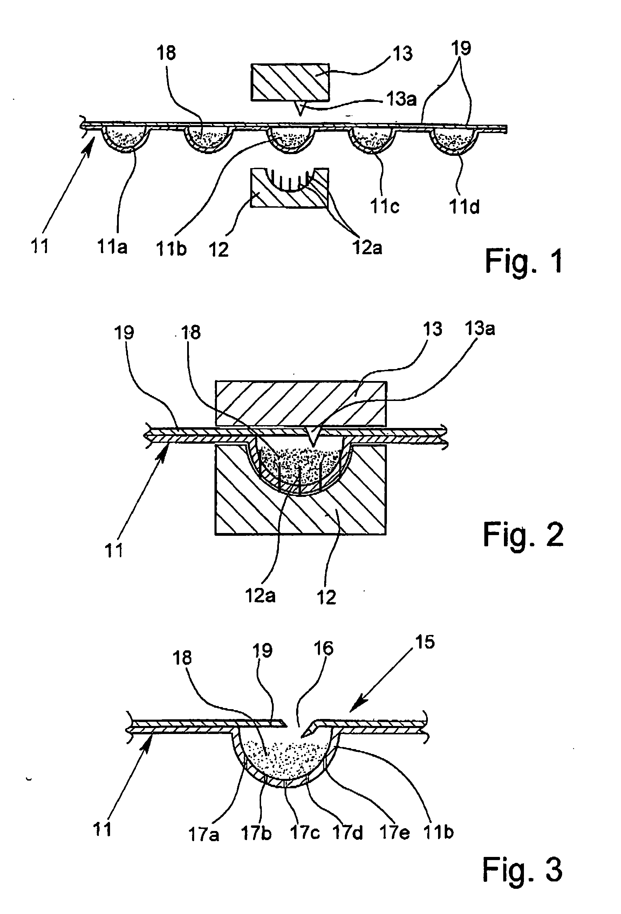

In Figure 1 a blister strip 11 has blister 11a to 11d. The strip 11 has lidding 19. The strip 11 is arranged to be moved so that one by one the blisters are in line with piercer 12 with pins 12a and piercer 13 with edge 13a.

In Figure 2 the piercer 12 and 13 are in their punching positions.

In Figure 3 a pierced blister 15 has punched hole 16 and pierced holes 17a to 17e. Ton empty blister and remove powder 18 air or gas is passed through holes 16 and 17.

Preferably, the first piercer 13 and/or the second piercer 12 are moveable.

Preferably, the first piercer 13 and the second piercer 12 are moveable relative to each other and/or relative to the blister strip 11 and/or blister 11 a-d, 15.

Preferably, each blister contains a dose of a medicament and/or powder 18.

Preferably, a or each blister is formed by a preferably bowl-like body 11a, 11b, 11c, 11d covered or closed by 11d 19.

In general, the blisters 11 a-d, 15 are opened from or on opposing sides, either by the same means or processes (here by piercing) or by different means or processes.

Not all possible embodiments are shown in the drawings. It must be understood that other embodiments are possible based on the invention.

This invention is concerned with dry powder inhalers for the delivery of drugs to the lungs. The present invention relates to an inhaler for discharging a medicament or inhalation formulation in the form of powder. The powder is discharged preferably by means of a gas or air stream flowing through the chamber to entrain the powder and to generate or form a powder spray for inhalation.

Object of the present invention is in particular to improve piercing of blisters and/or to improve discharge characteristics of blisters or inhalers.

The above object is achieved by independent claim 1. Preferred embodiments are subject of the subclaims. In particular, further aspects and features of the present invention will be apparent from the claims.

## Claims

1. Inhaler with a blister piercing mechanism for piercing a blister (11a-d, 15), the blister piercing mechanism comprising:
a first piercer (13) for making at least one hole in a lid of the blister; and
a second piercer (12) to make at least one hole (17a-e) in a body of the blister (11a-d, 15),
**characterized in that**
the second piercer (12) is arranged or designed such that more than four holes (17a-e) arc made in the body and evenly spaced out over at most of the body, to evenly distribute the air drawn or forced into the blister (11a-d, 15) over the entire blister (11a-d, 15)
the first piercer (13) piercing only the lid of the blister,
the second piercer (12) piercing only the body of the blister

2. Inhaler according to claim 1, **characterized in that** the first and second piercer (12, 13) are arranged opposite to each to other and/or on opposite sides of the blister (11a-d, 15).

3. Inhaler according to one of the previous claims, **characterized in that** the second piercer (12) has a preferably concave form adapted to the outer contour or form of the body of the blister (11a-d, 15).

4. Inhaler according to one of the previous claims, **characterized in that** the second piercer (12) comprises multiple sharp pins or needles (12a).

5. Inhaler according to one of the previous claims, **characterized in that** the second piercer (12) is arranged or designed such that 5 to 20 holes (17a-e) are made in the body.

6. Inhaler according to one of the previous claims, **characterized in that** the inhaler is a dry powder inhaler.

7. Inhaler according to one of the previous claims, **characterized in that** the inhaler is an active inhaler.

8. Inhaler according to one of the previous claims, **characterized in that** the inhaler comprises a gas or air pump for generating a gas or air flow to release powder (18) from a pierced blister.

9. Inhaler according to one of the previous claims, **characterized in that** the inhaler is a passive inhaler.

10. Inhaler according to one of the previous claims, **characterized in that** the inhaler comprises a blister (11a-d, 15).

11. Inhaler according to one of the previous claims, **characterized in that** the inhaler comprises a blister strip (20) with multiple blisters (11a-d, 15) which can be opened preferably one after the other by means of the blister piercing mechanism.

12. Inhaler according to one of the previous claims, **characterized in that** the inhaler or its blister piercing mechanism is designed or contructed such that a blister (11a-d, 15) or blister strip (11) can be opened from or on opposite sides.

## Patentansprüche

1. Inhalator mit einem Blisterdurchstechmechanismus zum Durchstechen eines Blisters (11a-d, 15), wobei der Blisterdurchstechmechanismus aufweist:
eine erste Durchstechvorrichtung (13) zur Herstellung mindestens eines Lochs in einem Deckel des Blisters und
eine zweite Durchstechvorrichtung (12) zur Herstellung mindestens eines Lochs (17a-e) in einem Körper des Blisters (11a-d, 15),
**dadurch gekennzeichnet,**
**dass** die zweite Durchstechvorrichtung (12) so angeordnet oder ausgeführt ist, dass mehr als vier Löcher (17a-e) in dem Körper hergestellt und über den Großteil des Körpers gleichmäßig beabstandet werden, um die in den Blister (11a-d, 15) gesaugte oder gedrückte Luft über den gesamten Blister (11a-d, 15) gleichmäßig zu verteilen,
wobei die erste Durchstechvorrichtung (13) nur den Deckel des Blisters durchsticht,
wobei die zweite Durchstechvorrichtung (12) nur den Körper des Blisters durchsticht.

2. Inhalator nach Anspruch 1, **dadurch gekennzeichnet, dass** die erste und die zweite Durchstechvorrichtung (12, 13) einander gegenüber und/oder auf gegenüberliegenden Seiten des Blisters (11a-d, 15) angeordnet sind.

3. Inhalator nach einem der vorhergehenden Ansprüche, **dadurch gekennzeichnet, dass** die zweite Durchstechvorrichtung (12) eine vorzugsweise konkave Form aufweist, die an die Außenkontur oder Form des Körpers des Blisters (11a-d, 15) angepasst ist.

4. Inhalator nach einem der vorhergehenden Ansprüche, **dadurch gekennzeichnet, dass** die zweite Durchstechvorrichtung (12) mehrere scharfe Stifte oder Nadeln (12a) aufweist.

5. Inhalator nach einem der vorhergehenden Ansprüche, **dadurch gekennzeichnet, dass** die zweite Durchstechvorrichtung (12) so angeordnet oder ausgeführt ist, dass 5 bis 20 Löcher (17a-e) in dem Körper hergestellt werden.

6. Inhalator nach einem der vorhergehenden Ansprüche, **dadurch gekennzeichnet, dass** es sich bei dem Inhalator um einen Trockenpulverinhalator handelt.

7. Inhalator nach einem der vorhergehenden Ansprüche, **dadurch gekennzeichnet, dass** es sich bei dem Inhalator um einen aktiven Inhalator handelt.

8. Inhalator nach einem der vorhergehenden Ansprüche, **dadurch gekennzeichnet, dass** der Inhalator eine Gas- oder Luftpumpe zur Erzeugung eines Gas- oder Luftstroms zur Freigabe von Pulver (18) aus einem durchstochenen Blister aufweist.

9. Inhalator nach einem der vorhergehenden Ansprüche, **dadurch gekennzeichnet, dass** es sich bei dem Inhalator um einen passiven Inhalator handelt.

10. Inhalator nach einem der vorhergehenden Ansprüche, **dadurch gekennzeichnet, dass** der Inhalator einen Blister (11a-d, 15) aufweist.

11. Inhalator nach einem der vorhergehenden Ansprüche, **dadurch gekennzeichnet, dass** der Inhalator einen Blistertreifen (20) mit mehreren Blistern (11a-d, 15) aufweist, die mittels des Blisterdurchstechmechanismus vorzugsweise nacheinander geöffnet werden können.

12. Inhalator nach einem der vorhergehenden Anspruch, **dadurch gekennzeichnet, dass** der Inhalator oder sein Blisterdurchstechmechanismus so konzipiert oder ausgeführt ist, dass ein Blister (11a-d, 15) oder Blisterstreifen (11) von oder auf gegenüberliegenden Seiten geöffnet werden kann.

## Revendications

1. Inhalateur comprenant un mécanisme de perçage de coque pour percer une coque (11a-d, 15), le mécanisme de perçage de coque comprenant:
un premier perceur (13) pour pratiquer au moins un trou dans un couvercle de la coque; et
un deuxième perceur (12) pour pratiquer au moins un trou (17a-e) dans un corps de la coque (11a-d, 15);
**caractérisé en ce**
**que** le deuxième perceur (12) est agencé ou conçu de telle sorte que plus de quatre trous (17a-e) soient pratiqués dans le corps et soient uniformément espacés sur la majeure partie du corps, afin de distribuer de façon uniforme l'air qui est aspiré ou forcé dans la coque (11a-d, 15) sur la totalité de la coque (11a-d, 15),
le premier perceur (13) perce uniquement le couvercle de la coque,
le deuxième perceur (12) perce uniquement le corps de la coque.

2. Inhalateur selon la revendication 1, **caractérisé en ce que** les premier et deuxième perceurs (12, 13) sont agencés l'un en face de l'autre et/ou sur des côtés opposés de la coque (11a-d, 15).

3. Inhalateur selon l'une quelconque des revendications précédentes, **caractérisé en ce que** le deuxième perceur (12) présente de préférence une forme concave qui est adaptée au contour ou à la forme extérieur(e) du corps de la coque (11a-d, 15).

4. Inhalateur selon l'une quelconque des revendications précédentes, **caractérisé en ce que** le deuxième perceur (12) comprend de multiples broches ou aiguilles affûtées (12a).

5. Inhalateur selon l'une quelconque des revendications précédentes, **caractérisé en ce que** le deuxième perceur (12) est agencé ou conçu de telle sorte que 5 à 20 trous (17a-e) soient pratiqués dans le corps.

6. Inhalateur selon l'une quelconque des revendications précédentes, **caractérisé en ce que** l'inhalateur est un inhalateur à poudre sèche.

7. Inhalateur selon l'une quelconque des revendications précédentes, **caractérisé en ce que** l'inhalateur est un inhalateur actif.

8. Inhalateur selon l'une quelconque des revendications précédentes, **caractérisé en ce que** l'inhalateur comprend une pompe à gaz ou à air pour générer un écoulement de gaz ou d'air afin de libérer de la poudre (18) à partir d'une coque percée.

9. Inhalateur selon l'une quelconque des revendications précédentes, **caractérisé en ce que** l'inhalateur est un inhalateur passif.

10. Inhalateur selon l'une quelconque des revendications précédentes, **caractérisé en ce que** l'inhalateur comprend une coque (11a-d, 15).

11. Inhalateur selon l'une quelconque des revendications précédentes, **caractérisé en ce que** l'inhalateur comprend une bande de coques (20) comprenant de multiples coques (11a-d, 15) qui peuvent être ouvertes de préférence l'une après l'autre au moyen du mécanisme de perçage de coque.

12. Inhalateur selon l'une quelconque des revendications précédentes, **caractérisé en ce que** l'inhalateur ou son mécanisme de perçage de coque est conçu ou constitué de telle sorte qu'une coque (11a-d, 15) ou une bande de coques (11) puisse être ouverte à partir de ou sur des côtés opposés.
